# EUROPEAN PATENT SPECIFICATION

(11) **EP 0 475 478 B1**
(45) Date of publication and mention of the grant of the patent: **05.07.1995**
(21) Application number: 91202039.3
(22) Date of filing: 08.08.1991
(51) Int. Cl.: A61K 39/102, C07K 2/00

(54) **Haemophilus paragallinarum vaccine**
Haemophilus Paragallinarum-Impfstoffe
Vaccine contre l'haemophilus paragallinarum

(30) Priority: 05.09.1990 EP 90202358
(43) Date of publication of application: 18.03.1992
(73) Proprietor: Akzo Nobel N.V., 6824 BM Arnhem (NL)
(72) Inventor: Jacobs, Antonius Arnoldus Christiaan, NL-5995 PS Kessel (NL)
(74) Representative: Hermans, Franciscus G.M.

(56) References cited:
- DE-A- 2 906 996
- GB-A- 1 324 619
- AVIAN DISEASES, vol. 33, 1989, College Station, TX (US); P.J. BLACKALL et al., pp. 168-173/
- BERGEY'S MANUAL OF SYSTEMATIC BACTERIOLOGY, vol. 1, 1987, Williams & Wilkins, Baltimore, MD (US); p. 568/

## Description

The present invention is concerned with a vaccine for the protection of poultry against Haemophilus paragallinarum infection, Haemophilus paragallinarum antigenic material, antibody or antiserum specific for said material and methods for the preparation of a Haemophilus paragallinarum vaccine.

Haemophilus paragallinarum (H. paragallinarum) causes an infection of the upper respiratory tract of chickens called infectious coryza (IC). The most prominent features are involvement of nasal passages and sinuses with a serous to mucoid nasal discharge, facial oedema and conjunctivitis. Infections of the lower respiratory tract, e.g. air sacs and lungs may occur. Although morbidity is high, natural mortality is relatively low. Frequently IC causes an increased number of culls, stunted growth and reduction in egg production resulting in high economic losses. Moreover, IC is usually more severe and prolonged, with resulting increased mortality when complicated with other agents such as fowl pox, Mycoplasma gallisepticum, infectious bronchitis, Pasteurella and infectious laryngotracheitis. Birds recovered from IC often remain reservoirs of infection and serve to maintain the infection in the flock.

At present for the vaccination against IC use is made of whole bacteria. These vaccines can be based on live, preferably attenuated strains, or inactivated virulent strains. However, attenuated live vaccines always involve the risk of inoculating animals with inadequately attenuated pathogenic bacteria which causes disease in the inoculated animal and possible spread of the disease in the flock. In addition attenuated bacteria may revert to a virulent state.

Inactivated vaccines also suffer from a number of drawbacks, e.g. the side-effects which can occur after administration of whole bacteria as a result of the presence of cell wall material, such as lipopolysaccharide (LPS).

Three distinct serotypes of H. paragallinarum can be distinguished, i.e. serotype A, B or C. Within each serotype a cross-protection based relationship exists between the various H. paragallinarum strains. However, birds vaccinated with a H. paragallinarum strain belonging to a specific serotype are not protected against infection of H. paragallinarum strains belonging to the other serotypes, i.e. immunity is type-specific. Hence, for a sufficient protection against IC preferably birds have to be immunized with a vaccine containing immunogenic material derived from several H. paragallinarum serotypes.

Beside the disadvantages of H. paragallinarum vaccines based on whole bacteria mentioned above an additional drawback of such a vaccine is that said vaccine containing whole bacteria of the B or C serotype only partially protects the vaccinated birds against subsequent infection with virulent strains of the B or C serotype.

A filamentous haemagglutinin of H. paragallinarum serotype A has been isolated and characterized by Iritani et al. (Am.J.Vet.res., 41 (1980), 2114 and German Pat. Applic. DE-A-2 906996 (Shionogi & Co., LTD.)). This filamentous haemagglutinin has protective activity against infection with H. paragallinarum serotype A strains.

However, Iritani's method for the isolation of the protective component of H. paragallinarum serotype A, i.e. releasing the protective activity from the cells by use of trypsin and isolating the carrier of said protective activity thereafter, is not applicable for serotype C, as according to this isolation procedure no protective component could be isolated from H. paragallinarum strains of this serotype.

A new method for the isolation of a protective fraction of H. paragallinarum has now been found which can be applied to all serotypes of H. paragallinarum.

Said method results in a new purified membraneous fraction of H. paragallinarum cells comprising a protein of 38 ± 3 kD, measured in SDS-PAGE (as outlined herein), which is highly protective against H. paragallinarum infection in poultry and which is essentially free from cells and filamentous material.

According to the present invention a subunit vaccine, i.e. a vaccine comprising only the necessary and relevant components for protection can be prepared which is derived from a membraneous fraction of H. paragallinarum cells comprising the protein of about 38 kD (38 kD protein enriched membraneous fraction). Chickens immunized with such a vaccine are well protected against homologous challenge with a virulent H. paragallinarum strain.

An even more complete protection can be achieved if a vaccine derived from an outer-membrane fraction of H. paragallinarum cells enriched in a 38 kD outer-membrane protein (OMP) is administered to chickens (38 kD OMP preparation).

This purified 38 kD OMP preparation is essentially free from cells and filamentous material.

The vaccine according to the invention can be further characterized in that the protective activity is
- sensitive to heat,
- sensitive to trypsin treatment, and
- sensitive to proteinase-K treatment.

A vaccine according to the invention can also comprise a fragment of said 38 kD OMP which still has immunizing properties, i.e. an immunological equivalent.

The 38 kD protein enriched membraneous fraction can be obtained by culturing H. paragallinarum bacteria in medium under conditions promoting expression of the 38 kD OMP, sedimentation of the cell membrane fraction following disruption of H. paragallinarum cells, e.g. enzymatically or mechanically (for example by sonication, grinding or french press), whereafter the 38 kD protein enriched membraneous fraction can be isolated, e.g. by centrifuging.

The 38 kD OMP preparation can be isolated by purifying the membraneous fraction mentioned above further into inner and outer membranes, e.g. by density gradient sedimentation or by differential solubilization of the inner membrane by detergents such as Triton^{(R)} X-100 or Sarcosine, followed by centrifugation
Another method for isolating above-noted fraction or preparation can be applied using antiserum (e.g. monoclonal antibodies) specific for the 38 kD OMP. A membrane extract of H. paragallinarum can be applied to a column substrate which contains said specific antiserum for the 38 kD OMP, separating the adsorbed fraction of the extract from the unadsorbed material, and subsequently releasing the adsorbed fraction from the column substrate.

If desired, the 38 kD OMP can be purified to homogenity by methods known in the art, e.g. solubilizing the 38 kD OMP from said 38 kD OMP preparation by extraction with one or more detergents followed by further purification, e.g. by ion exchange or molecular sieve chromatography, under conditions which do not affect the protective properties of the 38 kD OMP.

The purified 38 kD protein enriched fractions can suitably be isolated from H. paragallinarum strains from all available serotypes, e.g. serotypes A, B and C or corresponding serotypes known in the art.

The 38 kD OMP to be incorporated into a vaccine according to the invention can be obtained by chemical synthesis, purification from H. paragallinarum cells or by recombinant technology.

In the latter case nucleic acid sequences encoding above-mentioned protein or fragments thereof can for example be identified by screening a genomic H. paragallinarum DNA bank for individual clones comprising said sequences, e.g. by using a specific reaction with polyclonal or monoclonal antibodies elicited against the 38 kD OMP. The nucleic acid sequences can be ligated to various expression effecting DNA sequences, resulting in a so called recombinant nucleic acid molecule which can be used for the transformation of a suitable host. Such hybrid DNA molecules can for example be derived from plasmids or from nucleic acid sequences present in viruses. The host cell can be of prokaryotic origin, e.g. bacteria or eukaryotic origin such as mammalian cells. The transformed host cells can be used to produce the 38 kD OMP whereafter said protein or 38 kD OMP containing material can be isolated and subsequently incorporated into a vaccine according to the invention.

In another embodiment a live vector vaccine can be prepared comprising non-pathogenic micro-organisms, e.g. viruses or bacteria containing the nucleic acid sequence encoding the 38 kD OMP or fragment thereof cloned into the micro-organisms.

A vaccine according to the invention is derived from a 38 kD protein enriched membraneous fraction or 38 kD OMP preparation of at least one serotype of H. paragallinarum, i.e. vaccines comprising said fraction or preparation, or vaccines comprising immunological equivalents of the 38 kD OMP are within the scope of the invention.

Furthermore, a vaccine according to the invention may be in the form of a recombinant DNA vaccine as outlined above.

Preferably, a vaccine according to the present invention contains more than one 38 kD enriched membraneous fraction or 38 kD OMP preparation derived from H. paragallinarum strains of different serotypes. The most complete protection against H. paragallinarum infection is obtained if a vaccine is administered to birds which contains said membraneous fractions or preparations derived from H. paragallinarum strains of serotype A, B and C. Such a trivalent vaccine is a prefered embodiment of the present invention. A trivalent vaccine according to the invention completely protects birds vaccinated with said vaccine against H. paragallinarum infection as opposed to vaccines hitherto used based on whole bacteria.

Apart from the H. paragallinarum material carrying the protective activity a vaccine according to the invention may also contain an aqueous medium or a water containing suspension, often mixed with other constituents, e.g. in order to increase the activity and/or shelf life. These constituents may be salts, PH buffers, stabilizers (such as skimmed milk or casein hydrolysate), emulsifiers, adjuvants to improve the immune response (e.g. mineral oils, muramyl dipeptide, aluminium hydroxide, saponin, polyanions and amphipatic substances) and preservatives such as thimerosal.

A vaccine according to the invention may additionally contain also immunogenic material derived from other bacteria or viruses infectious to poultry, e.g. Mycoplasma, Newcastle Disease Virus and Infectious Bronchitis Virus.

Preferably the vaccine is administered parenterally, for example subcutaneously or intramuscularly. The vaccine may be administered in this manner both for the active immunization of the vaccinated birds and to laying birds for the passive, immunization of the offspring thereof. In immunized laying birds, the antibodies raised in them will, of course, be introduced into the yolks of their eggs and therefore subsequently in the hatched chicks.

Both the composition of the vaccine and the vaccination system can be varied and depend on the type of animal to be protected, the age and the weight of the animal, the desired duration of the protection, the method of administration and on the question of whether active immunization or passive immunization by means of maternal antibodies is desired. The optimally effective quantity of the active component in the vaccine is approximately 0.1 - 100 »g per dose for parenteral, e.g. intramuscular or subcutaneous vaccination of poultry. The most preferred dose ranges between 0.3 and 1.0 »g protein.

The above described active immunization against H. paragallinarum primarily will be applied as a protective treatment in healthy birds. It goes without saying that birds already infected by H. paragallinarum can be treated with antibodies directed against a membraneous fraction or preparation according to the invention.

Antiserum directed against a membrane fraction according to the present invention can be prepared by immunizing birds with an effective amount of said fraction, preferably in the presence of an adjuvant. If desired the animals can be boostered with a second vaccination to elicit an appropriate immune response. Thereafter the animals are bled and antiserum can be prepared.

The antiserum or antibodies can be mixed with a pharmaceutically acceptable carrier.

Polyclonal antiserum specific for the membraneous fraction derived from a H. paragallinarum strain of a specific serotype can be prepared by incubating antiserum elicited against said membraneous fraction, with a mixture of membraneous fractions of H. paragallinarum strains of the other serotypes. Antibodies not specific for said H. paragallinarum membraneous fraction will adsorb to the added H. paragallinarum fractions and can thus be separated from the incubation mixture resulting in an antiserum preparation specific for a H. paragallinarum membraneous fraction of a certain serotype.

Monoclonal antibodies directed against a membraneous fraction or preparation according to the invention can also be used for the passive immunizing of birds infected with H. paragallinarum. Said monoclonal antibodies can be produced by methods known in the art e.g. by immunizing mice with a membraneous fraction according to the invention, immortalizing mouse spleen cells and selecting hybridomas producing useful antibodies.

Above-mentioned antisera and monoclonal antibodies can also be used for the immunological diagnosis of birds infected with H. paragallinarum bacteria.

### Example 1

### Purification of H. paragallinarum membrane fractions and immunization of chickens

Bacterial strains were streaked out on chocolate agar, incubated for 24 h at 37 ^{o}C in an atmosphere in which a candle was allowed to burn out and then cultured in TPB supplemented with 0.01% NADH.

230 ml of a concentrated H-18 (serotype C) cell suspension (containing 4 x 10¹⁰ cells per ml of 40 mM PBS + 0.01% thimerosal) was ultrasonicated for 6 min. at 4 ^{o}C and 100 Watt, using a Branson B12 Sonifier (30 sec. periods of sonication were alternated by 30 sec. of cooling periods). Subsequently, the unbroken cells were spun down (10 min., 5000 x g) and 20 ml of the supernatant was emulsified in mineral oil water-in-oil adjuvant (W/O). This vaccine is called SUP-USO (C). The remaining 210 ml supernatant was centrifuged for 1 hour at 167,000 x g in an ultracentrifuge, to purify the total membrane fraction from the soluble fraction.

The membrane pellet (brownish colour) was resuspended in 60 ml 50 mM Tris-HCl, pH 8.0. 10 ml of this suspension was diluted 3 x with the same buffer and emulsified in W/O adjuvant. This vaccine is called 38 kD protein enriched membraneous fraction (C). The remaining 50 ml of the suspension was 1:1 mixed with 50 mM Tris-HCl, pH 8.0 + 2% Sodium-N-Lauroyl sarcosine and incubated for 2 h at room temperature, to solubilize the inner membranes. Subsequently, the mixture was again centrifuged for 1 hour at 167,000 x g to separate the solubilized inner membranes from outer membranes.

The pellet was suspended in 50 ml 50 mM Tris-HCl, pH 8.0. Part of this suspension was then diluted 3 x in the same buffer and emulsified in W/O adjuvant. This vaccine is called 38 kD OMP preparation (C).

The protective rate of the different purified fractions emulsified in W/O adjuvant is shown in Table 1.

Commercial Isabrown chickens were vaccinated once with 0.5 ml of the different vaccines at 10 weeks of age, and homologously challenged four weeks later with H. paragallinarum strain H-18 (serotype C). For each vaccine, 6 chickens were used.

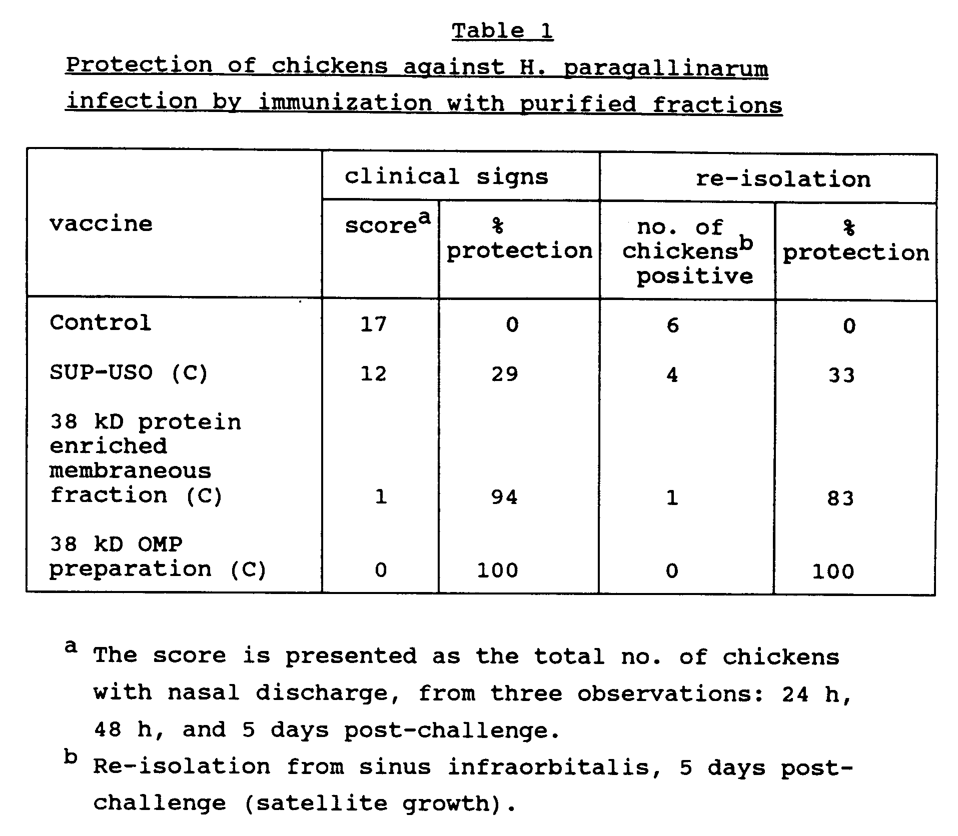

As is evident from Table 1, the purified membraneous fraction of H. paragallinarum and especially the purified 38 kD OMP preparation (C) appeared highly protective, presumably due to an optimal immune response evoked by said fraction.

For the preparation of a monovalent 38 kD OMP preparation (A) vaccine, strain 083 (A) was streaked out on chocolate agar and incubated for 24 hours at 37 ^{o}C in an atmosphere in which a candle was allowed to burn out and then cultured in TPB supplemented with 0.01% NADH. After incubation at 37 ^{o}C for 24 hours, the cells were spun down and purified 38 kD outer membrane protein preparation (A) was isolated exactly as described for the 38 kD OMP preparation (C).

Isolated OMP (A) was emulsified in W/O adjuvant to make vaccines containing 10 »g/0.5 ml and 1 »g/0.5 ml. To test these vaccines, commercial Isabrown chickens (10 weeks old) were vaccinated intramuscularly (brest) with a single 0.5 ml dose. At day of vaccination and 4 weeks post vaccination blood samples were taken and sera tested for hemagglutination-inhibition (HI) according to standard methods antibodies (indicative for protection).

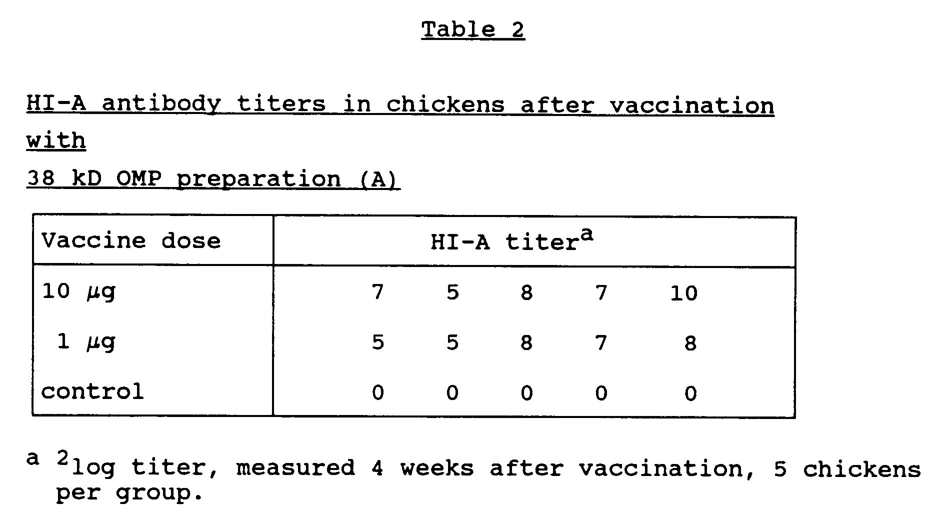

From the results (Table 2) it can be concluded that 100% of the chickens vaccinated with the 10 »g and 1 »g dose, would have been protected (HI-A > 2⁰).

Monovalent 38 kD OMP preparation (B) was prepared exactly as described above except that strain Spross (B) was cultured and used as starting material for the 38 kD OMP preparation isolation.

### Example 2

### Protection of chickens immunized with a trivalent vaccine

In an experiment a trivalent subunit vaccine (in W/O adjuvant) is prepared and protection is tested against challenge with strains of serotype A, B or C.

Furthermore, a monovalent H-18 (C) vaccine is tested against heterologous challenge with strain 083 (A) and strain Spross (B). The 38 kD OMP preparations of strains 083 (A), Spross (B) and H-18 (C) used for the trivalent subunit vaccine were purified exactly as described in Example 1. The trivalent vaccine contained 100 »g of each protein per dose of 0.5 ml. Monovalent H18 (C) vaccine is identical to the 38 kD OMP preparation vaccine used in Example 1.

Commercial Isabrown chickens were vaccinated once with 0.5 ml of vaccine at 10 weeks of age and challenged four weeks later. For each challenge group 7 chickens were used.

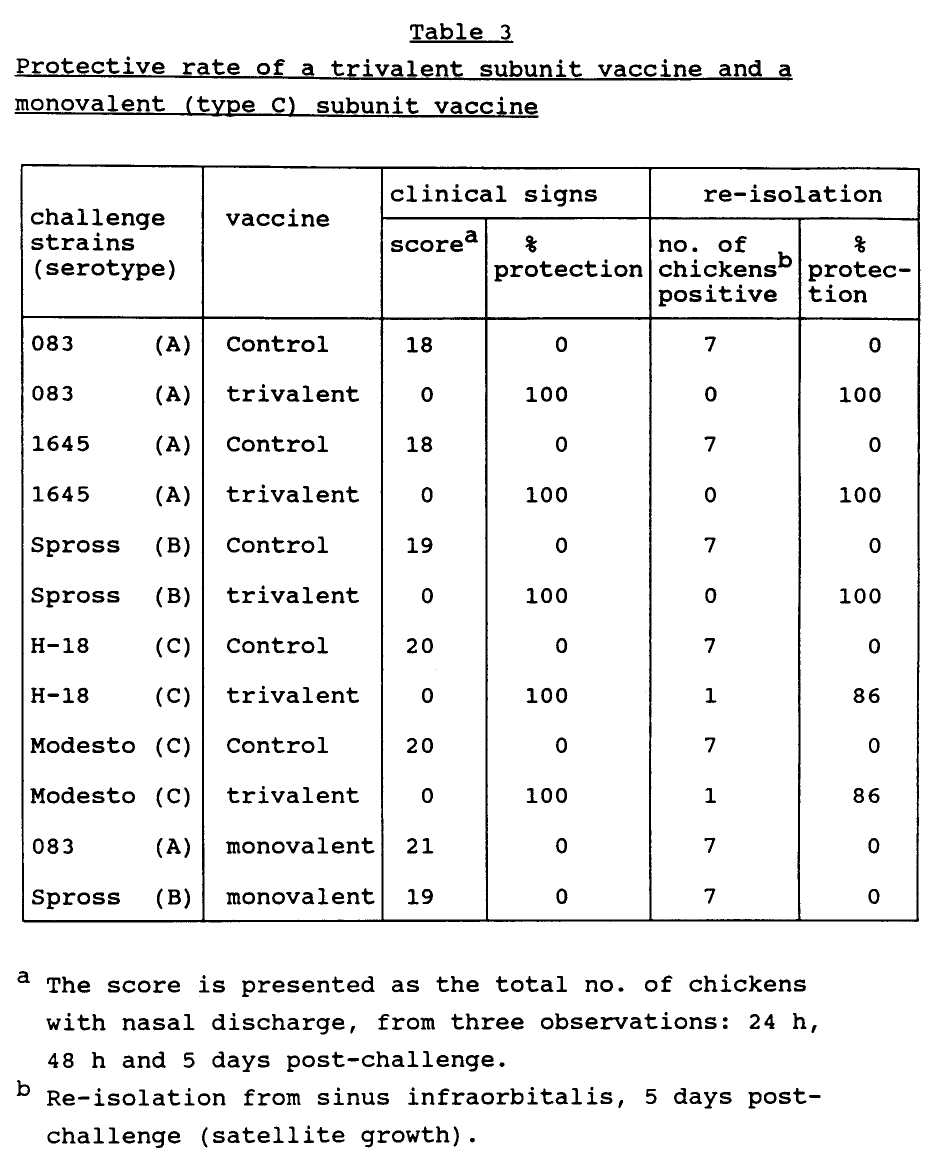

From Table 3 it appears that only homologous protection is induced after vaccination with the monovalent vaccine H-18 (C). The trivalent vaccine protected completely against challenge with all strains tested.

### Example 3

### Characterization of 38 kD protein enriched membraneous fractions

Purification of the 38 kD protein enriched membraneous fractions and 38 kD OMP preparations derived from serotype A, B and C strains of H. paragallinarum (strain 083, strain Spross and strain H-18, respectively) was carried out as described in Example 1.

Purified preparations were run in SDS-PAGE by the method of Laemmli (Nature (1970), 227, 680-685).

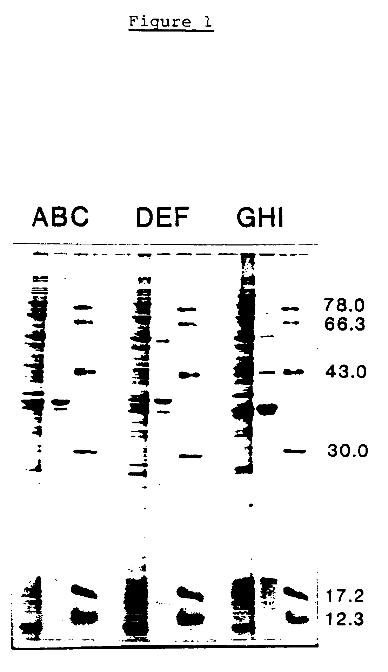

The SDS-PAGE with CBB staining of the purified preparations revealed an about 38 kD band as the dominant protein in the OMP preparations. (Figure 1). Figure 1 demonstrates the SDS-PAGE gelelectrophoresis profiles of different preparations i.e. SUP-USO and 38 kD OMP preparations, respectively, derived from strain 083 (A) (lanes A-B), Spross (B) (lanes D-E) and H-18 (C) (lanes G-H).
Molecular weight marker proteins are in lanes C, F and I.

| | |
|---|---|
| Cytochrome C, equine | 12.3 kD |
| Myoglobine, equine | 17.2 kD |
| Carbonic anhydrase, bovine | 30.0 kD |
| Ovalbumine, hen egg | 43.0 kD |
| Albumine, bovine serum | 66.3 kD |
| Ovotransferrin, hen egg | 78.0 kD |

In order to determine further characteristics of the protective activity of the 38 kD OMP preparations, these preparations were purified from strain 083 (A), Spross (B) and H-18 (C) as described in Example 1 and subjected to heat treatment and enzymatic digestion (trypsine and proteinase-K).
Trypsin splits proteins behind positively charged amino acid residues (i.e. lysin, arginin). Proteinase-K is a strong enzyme and splits most proteins into small peptides. Heating to 100 ^{o}C, leaves the amino acid chain intact but results in (mostly irreversible) unfolding of tertiary and quaternary structure of most proteins. For the enzymatic digestions 2 mg of protein was incubated with 200 »g of enzyme in 1 ml 40 mM PBS, 1.5 hours at 37 ^{o}C. Control preparations were treated in the same way except that the enzyme was left out. To obtain heat denatured proteins, 2 mg of protein in 1 ml 40 mM PBS was heated to 100 ^{o}C for 10 minutes.
Vaccine compositions were prepared resulting in a O/W emulsion comprising 50 »g protein per 0.5 ml dose and were tested against homologous challenge. Commercial Isabrown chickens (10 weeks old) were vaccinated once and challenged 4 weeks later. For each challenge group 6 chickens were used (Table 4).

From the protection experiments it can be concluded that the protective activity is sensitive to heat and enzymatic treatment, as such treatments result in a strong reduction of the protective capacity for all three serotypes.

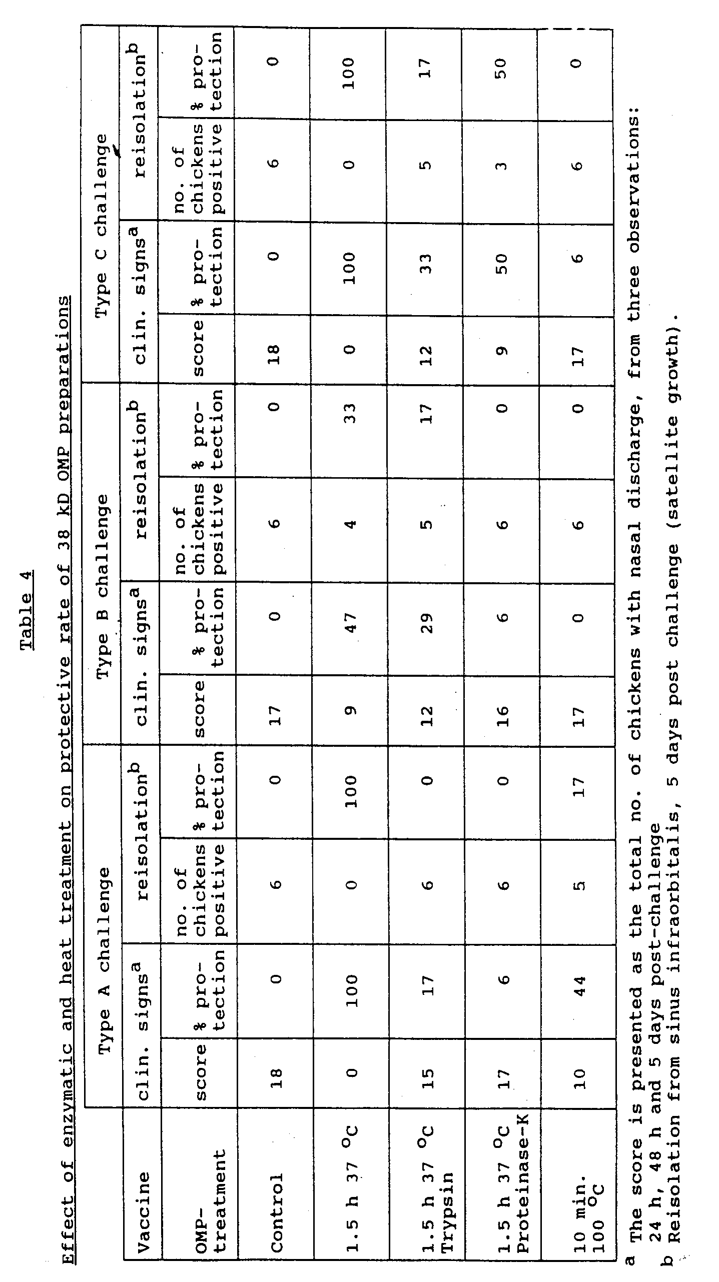

## Claims (Claims for the following Contracting State(s): BE, CH, DE, FR, GB, IT, LI, NL, SE)

1. Vaccine for the protection of poultry against Haemophilus paragallinarum infection, characterised in that the vaccine is essentially free from cells and is derived from a membraneous fraction of Haemophilus paragallinarum cells comprising a non-filamentous protein of 38 kD (+/- 3 kD) measured in SDS-PAGE, or an immunological equivalent of said protein.

2. Vaccine according to claim 1, characterized in that it is derived from an outer-membrane preparation having the 38 kD protein as a major protein component.

3. Vaccine according to claim 1 or 2, characterized in that it is derived from membraneous fractions of Haemophilus paragallinarum cells of at least two different serotypes.

4. Vaccine according to claim 3, characterized in that it is derived from membraneous fractions of Haemophilus paragallinarum cells of serotype A, B and C.

5. Vaccine according to claims 1-4, characterized in that it additionally contains at least one other micro-organism infectious to poultry or immunogenic material thereof.

6. Vaccine according to claims 1-5, characterized in that it comprises an adjuvant.

7. Haemophilus paragallinarum antigenic material, characterised in that it is a membraneous fraction of Haemophilus paragallinarum cells comprising a non-filamentous protein of 38 kD (+/- 3 kD) measured in SDS-PAGE, or an immunological equivalent of said protein, essentially free of cells.

8. Antigenic material according to claim 7, characterized in that it is an outer-membrane preparation having the 38 kD protein as a major protein component.

9. Antibody or antiserum immuno-reactive with the Haemophilus paragallinarum antigenic material according to claim 7 or 8.

10. Pharmaceutical preparation comprising an antibody or antiserum according to claim 9.

11. Method for the preparation of a vaccine according to claim 1, characterized in that Haemophilus paragallinarum bacteria are cultured in medium, whereafter membraneous material thereof comprising a non-filamentous protein of 38 kD (+/- 3 kD) is extracted therefrom and processed into a preparation with immunizing activity.

## Claims (Claims for the following Contracting State(s): ES, GR)

1. Method for the preparation of a vaccine for the protection of poultry against Haemophilus paragallinarum infection, characterised in that Haemophilus paragallinarum bacteria are cultured in medium, whereafter a membraneous fraction thereof, comprising a protein of 38 kD (+/- 3 kD) measured in SDS-PAGE, or an immunological equivalent thereof, essentially free of cells and filamentous material is prepared and mixed with a pharmaceutically acceptable carrier.

2. Method according to claim 1, characterised in that the membraneous fraction is an outer membrane preparation having the 38 kD protein as a major protein component.

3. Method according to claim 1 or 2, characterised in that the membraneous fraction is obtained from Haemophilus paragallinarum cells of at least two different serotypes.

4. Method according to claim 3, characterised in that the membraneous fraction is obtained from Haemophilus paragallinarum cells of serotypes A, B and C.

5. Method according to claims 1-4, characterised in that additionally at least one other micro-organism infectious to poultry or immunogenic material thereof is admixed.

6. Method according to claims 1-5, characterised that an adjuvant is admixed.

7. Method for the preparation of Haemophilus paragallinarum antigenic material, characterised in that a) Haemophilus paragallinarum bacteria are cultured and b) a membraneous fraction thereof comprising a protein of 38 kD (+/- 3 kD) measured in SDS-PAGE, or an immunological equivalent thereof, said fraction being essentially free from cells and filamentous material, is obtained by disruption of the cells followed by harvesting of the antigenic material.

8. Method according to claim 7, characterised in that the membraneous fraction is further purified into an outer membrane preparation having the 38 kD protein or an immunological equivalent thereof as a major protein component.

9. Method for the preparation of antibodies or antiserum immuno-reactive with the membraneous fraction of Haemophilus paragallinarum, characterised in that a) an effective amount of the antigenic material prepared according to claim 7 or 8 is administered to a suitable animal and b) antibodies or antiserum is isolated from the animal.

10. Method for the preparation of a pharmaceutical preparation comprising an antibody or antiserum, characterised in that an antibody or antiserum prepared according to claim 9 is mixed with a pharmaceutically acceptable carrier.

## Patentansprüche (Patentansprüche für folgende(n) Vertragsstaat(en): BE, CH, DE, FR, GB, IT, LI, NL, SE)

1. Impfstoff zum Schutz von Geflügel vor Infektion mit *Haemophilus paragallinarum*, dadurch gekennzeichnet, dass der Impfstoff im wesentlichen frei von Zellen ist und von einer Membranfraktion von *Haemophilus paragallinarum* Zellen stammt, welche ein nicht-filamentöses Protein von 38 kD (+/- 3 kD), gemessen in SDS-PAGE, oder ein immunologisches Äquivalent besagten Proteins umfasst.

2. Impfstoff gemäss Anspruch 1, dadurch gekennzeichnet, dass er von einem äusseren Membranpräparat, welches das 38-kD-Protein als Haupt-Proteinkomponente besitzt, stammt.

3. Impstoff gemäss Anspruch 1 oder 2, dadurch gekennzeichnet, dass er von Membranfraktionen von *Haemophilus paragallinarum*-Zellen von mindestens zwei verschiedenen Serotypen stammt.

4. Impfstoff gemäss Anspruch 3, dadurch gekennzeichnet, dass er von Membranfraktionen von *Haemophilus paragallinarum* Zellen der Serotypen A, B und C stammt.

5. Impfstoff gemäss Ansprüchen 1-4, dadurch gekennzeichnet, dass er zusätzlich mindestens einen für Geflügel infektiösen Mikroorganismus oder immunogenes Material davon enthält.

6. Impfstoff gemäss Ansprüchen 1-5, dadurch gekennzeichnet, dass er ein Adjuvans umfasst.

7. Antigenes Material von *Haemophilus paragallinarum*, dadurch gekennzeichnet, dass es eine Membranfraktion von *Haemophilus paragallinarum*-Zellen, die ein nichtfilamentöses Protein von 38 kD (+/- 3 kD), in SDS-PAGE gemessen, oder ein immunologisches Äquivalent besagten Proteins umfasst, im wesentlichen frei von Zellen ist.

8. Antigenes Material gemäss Anspruch 7, dadurch gekennzeichnet, dass es ein äusseres Membranpräparat mit dem 38-kD-Protein als hauptsächliche Proteinkomponente ist.

9. Antikörper oder Antiserum, das mit dem antigenen Material von *Haemophilus paragallinarum* gemäss Anspruch 7 oder 8 immunreaktionsfähig ist.

10. Pharmazeutisches Präparat, das einen Antikörper oder ein Antiserum gemäss Anspruch 9 umfasst.

11. Verfahren zur Herstellung eines Impfstoffes gemäss Anspruch 1, dadurch gekennzeichnet, dass *Haemophilus paragallinarum*-Bakterien in Medium kultiviert werden, wonach membranöses Material, das ein nicht-filamentöses Protein von 38 kD (+/- 3 kD) umfasst, daraus extrahiert wird und in ein Präparat mit immunisierender Aktivität verarbeitet wird.

## Patentansprüche (Patentansprüche für folgende(n) Vertragsstaat(en): ES, GR)

1. Verfahren zur Herstellung eines Impfstoffes zum Schutz von Geflügel vor Infektion mit *Haemophilus paragallinarum*, dadurch gekennzeichnet, dass *Haemophilus paragallinarum*-Bakterien in Medium kultiviert werden, wonach eine Membranfraktion davon hergestellt wird, die ein Protein von 38 kD (+/- 3 kD), in SDS-PAGE gemessen, oder ein immunologisches Äquivalent davon umfasst, die im wesentlichen frei von Zellen und filamentösem Material ist, und mit einem pharmazeutisch annehmbaren Trägerstoff gemischt wird.

2. Verfahren gemäss Anspruch 1, dadurch gekennzeichnet, dass die Membranfraktion ein Präparat der äusseren Membran mit dem 38-kD-Protein als hauptsächliche Proteinkomponente ist.

3. Verfahren gemäss Anspruch 1 oder 2, dadurch gekennzeichnet, dass die Membranfraktion von *Haemophilus paragallinarum*-Zellen von mindestens zwei verschiedenen Serotypen erhalten wird.

4. Verfahren gemäss Anspruch 3, dadurch gekennzeichnet, dass die Membranfraktion von *Haemophilus paragallinarum*-Zellen der Serotypen A, B oder C erhalten wird.

5. Verfahren gemäss Ansprüchen 1-4, dadurch gekennzeichnet, dass zusätzlich mindestens ein anderer für Geflügel infektiöser Mikroorganismus oder immunogenes Material davon beigemischt wird.

6. Verfahren gemäss Ansprüchen 1-5, dadurch gekennzeichnet, dass ein Adjuvans beigemischt wird.

7. Verfahren zur Herstellung von antigenem Material von *Haemophilus paragallinarum*, dadurch gekennzeichnet, dass a) *Haemophilus paragallinarum*-Bakterien kultiviert werden und b) eine Membranfraktion, die ein Protein von 38 kD (+/- 3 kD), in SDS-PAGE gemessen, oder ein immunologisches Äquivalent davon umfasst, wobei besagte Fraktion, die im wesentlichen frei von Zellen und filamentösem Material ist, durch Aufbrechen der Zelle, gefolgt vom Ernten des antigenen Materials erhalten wird.

8. Verfahren gemäss Anspruch 7, dadurch gekennzeichnet, dass die Membranfraktion weiter in ein Präparat der äusseren Membran mit dem 38-kD-Protein oder in ein immunologisches Äquivalent davon als hauptsächliche Proteinkomponente gereinigt wird.

9. Verfahren zur Herstellung von Antikörpern oder Antiserum, das mit der Membranfraktion von *Haemophilus paragallinarum* immunreaktiv ist, gekennzeichnet dadurch, dass a) eine wirkungsvolle Menge des antigenen Materials, das gemäss Anspruch 7 oder 8 hergestellt wird, einem geeigneten Tier verabreicht wird und b) Antikörper oder Antiserum aus dem Tier isoliert werden.

10. Verfahren zur Herstellung eines pharmazeutischen Präparats, das einen Antikörper oder ein Antiserum umfasst, dadurch gekennzeichnet, dass ein Antikörper oder ein Antiserum, das gemäss Anspruch 9 hergestellt wird, mit einem pharmazeutisch annehmbaren Trägerstoff gemischt wird.

## Revendications (Revendications pour l'(les) Etat(s) contractant(s) suivant(s): BE, CH, DE, FR, GB, IT, LI, NL, SE)

1. Vaccin pour la protection des volailles contre une infection par Haemophilus paragallinarum, caractérisé en ce que le vaccin est essentiellement exempt de formes cellulaires et est dérivé d'une fraction membranaire de cellules d'Haemophilus paragallinarum comprenant une protéine non filamenteuse de 38 kD (± 3 kD) tel que mesuré en SDS-PAGE, ou un équivalent immunologique de cette protéine.

2. Vaccin selon la revendication 1, caractérisé en ce qu'il dérive d'une préparation de membrane externe comportant la protéine de 38 kD en tant que composant protéique majeur.

3. Vaccin selon la revendication 1 ou 2, caractérisé en ce qu'il dérive de fractions membranaires de cellules de Haemophilus paragallinarum d'au moins deux sérotypes différents.

4. Vaccin selon la revendication 3, caractérisé en ce qu'il dérive de fractions membranaires de cellules d'Haemophilus paragallinarum de sérotypes A, B et C.

5. Vaccin selon les revendications 1 à 4, caractérisé en ce qu'il contient de plus au moins un autre micro-organisme infectieux à l'égard des volailles ou du matériel immunogène en dérivant.

6. Vaccin selon les revendications 1 à 5, caractérisé en ce qu'il comprend un adjuvant.

7. Matériel antigénique d'Haemophilus paragallinarum, caractérisé en ce qu'il correspond à une fraction membranaire de cellules d'Haemophilus paragallinarum comprenant une protéine non filamenteuse de 38 kD (± 3 kD) tel que mesuré en SDS-PAGE, ou un équivalent immunologique de cette protéine, essentiellement exempt de formes cellulaires.

8. Matériel antigénique selon la revendication 7, caractérisé en ce qu'il correspond à une préparation de membrane externe comportant la protéine de 38 kD en tant que composant protéique majeur.

9. Anticorps ou antisérum réagissant immunologiquement avec le matériel antigénique d'Haemophilus paragallinarum selon la revendication 7 ou 8.

10. Préparation pharmaceutique comprenant un anticorps ou un antisérum selon la revendication 9.

11. Procédé de préparation d'un vaccin selon la revendication 1, caractérisé en ce que les bactéries Haemophilus paragallinarum sont cultivées dans un milieu, puis le matériel membranaire en dérivant comprenant une protéine non-filamenteuse de 38 kD (± 3 kD) en est extrait et est formulé en une préparation présentant une activité immunisante.

## Revendications (Revendications pour l'(les) Etat(s) contractant(s) suivant(s): ES, GR)

1. Un procédé de préparation d'un vaccin pour la protection des volailles contre une infection par Haemophilus paragallinarum, caractérisé en ce que les bactéries d'Haemophilus paragallinarum sont cultivées dans un milieu, puis une fraction membranaire en dérivant, comprenant une protéine de 38 kD (± 3 kD) tel que mesuré en SDS-PAGE, ou un équivalent immunologique de cette dernière, essentiellement exempt de formes cellulaires et de matériel filamenteux est préparée et mélangée avec un support pharmaceutiquement acceptable.

2. Procédé selon la revendication 1, caractérisé en ce que la fraction membranaire est une préparation de membrane externe comportant la protéine de 38 kD en tant que composant protéique majeur.

3. Procédé selon la revendication 1 ou 2, caractérisé en ce que la fraction membranaire est obtenue à partir de cellules de Haemophilus paragallinarum d'au moins deux sérotypes différents.

4. Procédé selon la revendication 3, caractérisé en ce que la fraction membranaire est obtenue à partir de cellules d'Haemophilus paragallinarum de sérotypes A, B et C.

5. Procédé selon les revendications 1 à 4, caractérisé en ce que, de plus, au moins un autre micro-organisme infectieux pour les volailles ou du matériel immunogène en dérivant est mélangé.

6. Procédé selon les revendications 1 à 5, caractérisé en ce qu'un adjuvant est mélangé.

7. Procédé de préparation de matériel antigénique d'Haemophilus paragallinarum, caractérisé en ce que:
a) des bactéries d'Haemophilus paragallinarum sont cultivées; et
b) une fraction membranaire en dérivant comprenant une protéine de 38 kD (± 3 kD) tel que mesuré en SDS-PAGE, ou un équivalent immunologique en dérivant, ladite fraction étant essentiellement exempte de formes cellulaires et de matériel filamenteux, est obtenue par rupture des cellules suivie d'une récolte du matériel antigénique.

8. Procédé selon la revendication 7, caractérisé en ce que la fraction membranaire est davantage purifiée en une préparation de membrane externe comportant la protéine de 38 kD ou un équivalent immunologique en dérivant en tant que composant protéique majeur.

9. Procédé de préparation d'anticorps ou d'antisérum réagissant immunologiquement avec la fraction membranaire d'Haemophilus paragallinarum, caractérisé en ce que:
a) une quantité efficace du matériel antigénique préparé selon la revendication 7 ou 8, est administrée à un animal approprié; et
b) les anticorps ou l'antisérum est isolé à partir de l'animal.

10. Procédé de préparation d'une préparation pharmaceutique comprenant un anticorps ou un antisérum, caractérisé en ce que l'anticorps ou l'antisérum préparé selon la revendication 9 est mélangé avec un support pharmaceutiquement acceptable.
